# EUROPEAN PATENT APPLICATION

(11) **EP 4 062 901 A1**
(43) Date of publication of application: **28.09.2022**
(21) Application number: 22397502.0
(22) Date of filing: 25.03.2022
(51) Int. Cl.: A61K 8/9789, A61P 17/10, A61Q 19/00, A61Q 19/08

(54) **COSMETIC USE OF NORDIC BERRY INGREDIENTS FOR SUPPORTING A HEALTHY SKIN MICROBIOME**

(30) Priority: 26.03.2021 FI 20215349
(71) Applicant: Lumene Oy, 02780 Espoo (FI)
(72) Inventor: PESONEN, Terhi, 02780 ESPOO (FI); SAHRAMO, Elina, 02780 ESPOO (FI); PALMUJOKI, Ingela, 02780 ESPOO (FI); VÄNTTINEN, Kristofer, 02780 ESPOO (FI); MÄKINEN, Päivi, 02780 ESPOO (FI); ISOHANNI, Tiina, 02780 ESPOO (FI)
(74) Representative: Laine IP Oy

(57) **Abstract**

According to an example aspect of the present invention, there is provided the non-therapeutic use of an effective amount of spray-dried berry powder from Nordic berries as a cosmetic agent supporting a healthy skin microbiome, and a corresponding method. The method of the invention preferably comprises increasing the ratio of S. *epidermis* to C. *acnes* and S. *aureus* in human skin microbiome by applying on a human skin an effective amount of cosmetic composition comprising spray-dried lingonberry powder, spray-dried cloudberry powder or both.

## Description

### Field

The present invention relates to the field of cosmetic industry. In particular, the present invention relates to cosmetic use of an effective amount of spray-dried berry powder as an agent supporting a healthy skin microbiome. The invention also relates to a non-therapeutic method for increasing the ratio of commensal bacteria to harmful or pathogenic bacteria in human skin microbiome, in particular for increasing the ratio of *Staphylococcus epidermidis* to *Cutibacterium acnes (Propionibacterium acnes), Staphylococcus aureus* or other harmful or pathogenic bacteria, by applying on the skin a topical cosmetic composition comprising an effective amount of spray-dried berry powder from Nordic berries and a cosmetically acceptable carrier.

### BACKGROUND

Microbiome is generally defined as an "ecological community of commensal, symbiotic and pathogenic microorganisms influenced by environmental variables". Human skin is colonized by millions of bacteria, fungi and viruses that compose the skin microbiome. As human skin functions as the exterior interface of the human body with the environment, it acts as a physical barrier to prevent the invasion of foreign pathogens while providing a home to the commensal microbes.

A healthy human skin shows a very diverse and heterogeneous microbiome while in case of skin problems the balance between commensal microbes and pathogens is disturbed. Most bacteria living in the skin microbiome are harmless on healthy skin, but might cause diseases on unhealthy skin (Davis 1996). The skin microbiome is typically populated with a wide variety of micro-organisms and there are variations in the relative abundance of bacterial taxa populating dry, moist or sebaceous areas on the skin (Byrd et al. 2018). Dry skin areas, e.g. forearms and legs have the most diverse microbiota. These areas are dominated by *Propionibacterium, Corynebacterium, Staphylococcus, Streptococcus, Acinetobacter,* and many other bacterial species in lower amounts (San Miguel and Grice 2015). *Corynebacterium spp.* are common in moist areas of the skin e.g. axillary area (Callewaert et al. 2013).

The skin microbiome continues to evolve throughout life. It is affected by skin's condition, including the amount of water, pH, lipids, proteins, humectants, etc. The human skin microbiome is also affected by external environment and people we get in contact with, since we may also adopt bacteria from others. Moreover, the skin microbiome is also affected by the skin care products we use. Exposure to preservatives from cosmetics may kill microbes. Certain pH ranges of cosmetic products may favour growth for specific bacteria and reduce the growth of others.

Commensal bacteria are bacteria inhabiting the host for long-periods without causing harm or benefit, as opposed to harmful or pathogenic bacteria that may cause diseases. Prebiotics are typically carbohydrates that stimulate the growth of commensal bacteria, while probiotics generally include "good" bacteria that may prevent or reduce the growth of harmful or pathogenic bacteria.

*Staphylococcus epidermidis (S. epidermidis*) is one of the most common skin commensal bacteria. Colonisation of *S. epidermidis* is beneficial for the skin as several studies have shown that it can act as pro-biotic bacteria preventing the growth of harmful or pathogenic bacteria on the skin. S. *epidermidis* has been shown to inhibit biofilm formation of the pathogenic *Staphylococcus aureus* bacteria by secreting a serine protease (Iwase et al., 2010). In addition, Wang et al (2014) showed that *S. epidermidis* is able to ferment glycerol present naturally in skin to restrict the growth of *Propionibacterium acnes (Cutibacterium acnes),* skin bacteria which is known to cause acne vulgaris in skin.

*Cutibacterium acnes* is a normally harmless bacterium on the skin known to be associated with acne. This bacterium thrives on adolescent skin because of its increased fat content, which favours this bacterium. Acne (*Acne vulgaris*) is a common skin condition caused by excessive sebum secretion by the sebaceous glands or thickening of the stratum corneum of the top layer of the skin. In this case, the sebum cannot be excreted freely from the sebaceous gland, whereby the sebaceous gland may become inflamed. Sebum is an important nutritional factor for *Cutibacterium acnes.* When sebum is abundant on the skin, there are also many viable *C. acnes* bacteria.

In addition to the beneficial effects caused by colonization of *S. epidermidis,* colonisation of *C*. *xerosis* has also been shown to reduce the growth of pathogenic *Staphylococcus aureus* or *Pseudomonas aeruginosa* when grown in stimulated serum (Oates & McBain 2016).

It would thus be beneficial to find means to support the growth of commensal bacteria, such as *S. epidermidis,* and hence to limit the growth of harmful or pathogenic bacteria in human skin microbiome. In terms of cosmetic skin care, it would be particularly beneficial to achieve a good balance between *S. epidermidis* and *C. acnes* in human skin. A balanced skin microbiome is more capable to resist signs of aging, while also reducing dryness, redness and irritation of the skin. Therefore, prebiotics that would increase the amounts of *S. epidermidis* in the skin and reduce *C. acnes* bacteria are needed in cosmetic, non-therapeutic skin care.

Various berry-derived substances have been used in cosmetic compositions. EP 3365068 relates to cosmetic compositions containing spray-dried powder derived from cloudberry (*Rubus chamaemorus*) fruit for use in skin whitening. EP 1337224 describes cosmetic compositions containing cloudberry seed oil. EP 2914242 discloses a method for exploiting the press cake obtained when pressing juice from lingonberry (*Vaccinium vitis-idaea*)*,* thus producing ingredients which are useful in cosmetic compositions. US 8501246 relates to a method for treating a skin condition of a human by applying to the skin an effective amount of lingonberry extract. WO 2006/134583 suggests the use of a combination of lingonberry fruit and leaf extract in the manufacture of a cosmetic composition for treating various skin conditions. WO 2018/115582 A1 suggests that extracts obtained from ground berry press cakes or raspberry leaves by hydrothermal extraction exhibit antimicrobial activity.

However, the non-therapeutic use of spray-dried powder derived from Nordic berries, typically from cloudberry or lingonberry, in a cosmetic composition for increasing the ratio of commensal bacteria to harmful or pathogenic bacteria in human skin microbiome or the corresponding method have not been disclosed or suggested in prior art.

### SUMMARY OF THE INVENTION

The invention is defined by the features of the independent claims. Some specific embodiments are defined in the dependent claims.

The present invention is based on the finding that spray-dried berry powder from Nordic berries, particularly spray-dried lingonberry or cloudberry powder obtained from berries grown in the Nordic area, promotes the growth of commensal bacteria in relation to harmful or pathogenic bacteria of the human skin.

According to a first aspect of the present invention, there is thus provided the non-therapeutic, cosmetic use of spray-dried berry powder from Nordic berries in a cosmetic composition for increasing the ratio of commensal bacteria to harmful or pathogenic bacteria in human skin microbiome.

According to a second aspect of the present invention, there is provided a non-therapeutic cosmetic method for increasing the ratio of commensal bacteria to harmful or pathogenic bacteria in human skin microbiome, the method comprising applying on the skin a topical cosmetic composition comprising an effective amount of spray-dried berry powder from Nordic berries, in particular from lingonberry (*Vaccinium vitis-idaea*)*,* cloudberry (*Rubus chamaemorus*)*,* or both.

Embodiments of the invention comprise the above mentioned non-therapeutic method and use for increasing the ratio of *Staphylococcus epidermidis* to harmful or pathogenic bacteria in human skin microbiome, in particular for increasing the ratio of *Staphylococcus epidermidis* to *Cutibacterium acnes (Propionibacterium acnes), Staphylococcus aureus, Pseudomonas aeruginosa* or other harmful or pathogenic bacteria of human skin or any combinations thereof.

Considerable advantages are obtained by the invention. First, when the growth of harmful or pathogenic bacteria is limited and the skin microbiome is balanced, the skin is able to effectively act as a physical barrier to prevent the skin and thus the human body from the undesired cosmetic effects of harmful bacteria.

Second, a healthy and balanced skin microbiome helps to keep the surface of skin smooth and strong, while also reducing dryness, redness and irritation of the skin. Further, a strong and balanced skin microbiome is more capable to resist signs of aging.

Third, various skin conditions like mild acne and mild forms of atopic skin can be alleviated by increasing the ratio of commensal bacteria in human skin. For example, atopic skin is typically rich in *S. aureus.* Balancing the skin microbiome by increasing the ratio of *S. epidermidis* to *S. aureus* may thus alleviate the symptoms of atopic skin, such as dry skin itching. Mild forms of atopic, dry skin can benefit from cosmetic products balancing the skin microbiome.

Moreover, a balanced skin microbiome provides protection from external aggressors, such as air pollution. Air pollutants may cause the complexion appear uneven and dull and the skin feels uncomfortable.

In some embodiments, the prebiotic effect of Nordic berry powders may also alleviate undesirable skin odours caused by unbalanced skin microbiome, typically in the underarm area.

Further features and advantages of the present technology will appear from the following description of some embodiments.

### BRIEF DESCRIPTION OF THE DRAWINGS

FIGURES 1A and 1B illustrate how lingonberry spray-dried powder (45%) at a dose of 2 mg/ml increases the ratio of *S. epidermidis* at 24h and 48h while the ratio of *C. acnes* and *S. aureus* decreases compared to the initial state and the control.
FIGURES 2A and 2B illustrate how cloudberry spray-dried powder (30%) at a dose of 15 mg/ml increases the ratio of *S. epidermidis* at 24h and 48h while the ratio of *C. acnes* and *S*. *aureus* decreases compared to the initial state and the control.

### EMBODIMENTS

### DEFINITIONS

In the present context, the term "Nordic berries" typically comprises lingonberry *(Vaccinium vitis-idaea),* members of *Rubus* genus, such as cloudberry *(Rubus chamaemorus),* raspberry (*Rubus idaeus*)*,* and Arctic bramble (*Rubus arcticus*)*,* as well as cranberry (*Vaccinium oxycoccus* / *Oxycoccus palustris*) and strawberry (*Fragararia vesca*)*,* in particularly lingonberry *(Vaccinium vitis-idaea),* cloudberry *(Rubus chamaemorus),* or both, grown in the Nordic countries. By definition, the Nordic countries include Denmark, Finland, Iceland, Norway, and Sweden, as well as Greenland, the Faroe Islands, the Åland islands and Svalbard archipelagos. In the present context, the term "Nordic berries" refers mainly to berries naturally growing in the Nordic countries without agriculture or gardening. However, to ensure diversity of Nordic nature, berries may also be cultured. The natural ingredients may thus originate from organic farming methods or from wild harvesting in compliance with national legislation.

In the present context, "commensal bacteria" refer to bacteria inhabiting the human skin microbiome for long periods without causing harm or benefit, as opposed to harmful or pathogenic bacteria that may cause disease. One of the most common commensal skin bacteria is *Staphylococcus epidermidis.* Also e.g. *Staphylococcus hominis* has proven beneficial to human skin (Byrd, 2018). However, it is also known that in certain contexts, bacteria that are ordinarily beneficial to their hosts may become harmful or pathogenic.

In the present context, "harmful or pathogenic bacteria" include such bacteria that are potential to cause harmful symptoms or disease on human skin, in particular when their population or share in the skin microbiome increases. Examples of typical harmful or pathogenic bacteria of human skin microbiome include but are not limited to *Cutibacterium acnes (Propionibacterium acnes), Staphylococcus aureus, Pseudomonas aeruginosa, Escherichia coli, Corynebacterium mastitidis,* and *Corynebacterium bovis,* in particular *Cutibacterium acnes (Propionibacterium acnes)* and *Staphylococcus aureus.*

An "effective amount" refers to an amount adequate to accomplish a desired, expected, or intended result.

It has been found that spray-dried berry powder from Nordic berries, particularly lingonberry or cloudberry powder obtained from berries grown in the Nordic area, promotes the growth of commensal bacteria in relation to harmful or pathogenic bacteria of the human skin.

Therefore, the present invention provides the non-therapeutic use of spray-dried lingonberry powder, spray-dried cloudberry powder, or both in cosmetic compositions for increasing the proportion of *Staphylococcus epidermidis* in relation to harmful or pathogenic bacteria of human skin.

In a normal situation, *S. epidermidis* is a major inhabitant of the skin, and in some areas it may make up more than 90 percent of the resident aerobic flora. Another species of *Staphylococcus* sp, namely *Staphylococcus aureus,* is extremely common (80 to 100 percent) on the skin of patients with certain dermatologic diseases, such as atopic dermatitis. Thus colonization by *S. aureus* on the skin is one of the factors that can worsen general condition of the skin, including atopic dermatitis.

Anaerobic diphtheroids (coryneforms) are most common in areas rich in sebaceous glands. Although the name *Corynebacterium acnes* was originally used to describe skin anaerobic diphtheroids, these are now classified as *Propionibacterium acnes* and as *P. granulosum. P. acnes* is seen more frequently than *P. granulosum* in acne lesions and is probably involved in acne pathogenesis (Davis, 1996).

*In vitro* studies have shown that spray-dried powders from Nordic berries, in particular spray-dried lingonberry powder and spray-dried cloudberry powder, increase the proportion of commensal bacteria, in particular *S. epidermidis,* in relation to harmful or pathogenic bacteria, typically *C. acnes* and *S. aureus.* This finding finds use in cosmetic applications, such as in a non-therapeutic cosmetic method for increasing the ratio of commensal bacteria to harmful or pathogenic bacteria in human skin microbiome.

Thus in one embodiment, cosmetic compositions comprising spray-dried lingonberry powder, spray-dried cloudberry powder, or both, together with a cosmetically acceptable carrier, are used for increasing the ratio of *Staphylococcus epidermidis* to *Cutibacterium acnes (Propionibacterium acnes), Staphylococcus aureus, Pseudomonas aeruginosa* or other harmful or pathogenic bacteria or any combinations thereof, by applying said compositions topically on the skin.

In another embodiment the cosmetic compositions comprising spray-dried lingonberry powder, spray-dried cloudberry powder, or both, together with a cosmetically acceptable carrier, are used for increasing the ratio of *S*. *epidermidis* to *C*. *acnes, S. aureus* or their combination.

The changes in the ratio of commensal bacteria to harmful or pathogenic bacteria are typically seen within 6 to 48 hours after applying the composition on the skin, preferably within 12 to 24 hour after applying the composition on the skin. As understood by the persons skilled in the art the composition can be applied on the skin several times, for example twice a day, during a period of several days or weeks, to achieve an optimal result.

More particularly, the cosmetic compositions comprising spray-dried lingonberry powder, spray-dried cloudberry powder, or both, together with a cosmetically acceptable carrier, may increase the ratio of *S. epidermidis* to the combined about of *C. acnes* and *S. aureus,* typically from 1:2.5 - 1:3 to about 3:1 - 10:1 within 48 hours.

In one embodiment, the cosmetic compositions comprising spray-dried lingonberry powder, spray-dried cloudberry powder, or both, together with a cosmetically acceptable carrier, may increase the ratio of *S. epidermidis* to the combined about of *C. acnes* and *S. aureus* from about 1:2.5 to at least 3:1 within 24 hours from applying the cosmetic composition on the human skin.

In a further embodiment, the cosmetic compositions comprising spray-dried lingonberry powder, spray-dried cloudberry powder, or both, together with a cosmetically acceptable carrier, are used for increasing the ratio of *S. epidermidis* to *S. aureus.*

Typically, the cosmetic composition comprises spray-dried lingonberry powder having a lingonberry concentration of at least 30% by weight, preferably at least 45% by weight. The amount of the spray-dried lingonberry powder in the cosmetic composition is 0.01 to 20 % by weight, preferably 0.05 to 10% by weight, more preferably 0.05 to 5% by weight.

Typically, the cosmetic composition comprises spray-dried cloudberry powder having a cloudberry concentration of at least 30% by weight, preferably at least 40% by weight. The amount of the spray-dried cloudberry powder in the cosmetic composition is typically from 0.01 to 20 % by weight, preferably from 0.05 to 10 % by weight, more preferably from 0.05 to 5% by weight.

In an embodiment of the invention, wherein the cosmetic composition comprises both spray-dried lingonberry powder and spray-dried cloudberry powder, their combined amount in the cosmetic composition is typically from 0.01 to 20% by weight, preferably from 0.05 to 10% by weight.

An embodiment of the invention is also a non-therapeutic, cosmetic method for increasing the ratio of commensal bacteria to harmful or pathogenic bacteria in human skin microbiome, wherein the method comprises applying on the skin a topical cosmetic composition comprising an effective amount of spray-dried berry powder from Nordic berries, in particular from lingonberry, cloudberry or both, and a cosmetically acceptable carrier.

In an embodiment, the method and the corresponding use comprise applying the topical composition on non-acneic and/or non-atopic skin.

In the method according to the invention the topical composition typically comprises spray-dried lingonberry powder, spray-dried cloudberry powder or both in an amount of 0.01 to 20% by weight, preferably from 0.05 to 10% by weight, wherein the spray-dried lingonberry powder has a lingonberry concentration of at least 45% by weight, and the spray-dried cloudberry powder has a cloudberry concentration of at least 30% by weight.

In a preferred embodiment of the invention, the spray-dried berry powder from Nordic berries is obtained by pressing juice from the berries, filtering the juice, mixing the juice with maltodextrin and then spray-drying the mixture.

### Spray-dried cloudberry powder

Spray-dried cloudberry powder is typically obtained by pressing the juice from cloudberry fruit, filtering and mixing the juice with maltodextrin, and then spray-drying the mixture. Cloudberries (*Rubus chamaemorus*) are collected from a wetland, typically in northern or eastern Finland. The berries are typically heat-treated and treated with pectinase, and the juice is pressed using a conventional presser. Subsequently, the juice may be filtered, pasteurized and concentrated. If necessary, the concentrate is diluted with water, followed by mixing with maltodextrin, for example in a ratio of 30% of cloudberry juice and 70% of maltodextrin (per weight) when 30% cloudberry powder is prepared. The mixture is spray-dried in a conventional spray-drier, by leading the concentrated mixture into the drying chamber as spray, whereby water is evaporated and the dried particles separated. The powder is cooled down, sieved out and packed for further use.

The composition of the spray-dried cloudberry powder (30%) typically consists of mainly carbohydrates 89 g/100g of which about 18g are sugars, while it also contains approximately 3% of dietary fiber. Vitamin C content varies but is typically at least about 60 mg/100g, preferably about 80-100 mg/100 g. Total content of polyphenolic compounds also varies but is typically at least about 2-2.5 mg/g, measured using Folin-Ciocalteau method. In the present context, spray-dried cloudberry powder typically has a total ellagitannin concentration of at least 11 mg/g dry matter and/or a phenolic acids content of at least 0.25 mg/g dry matter. Antioxidant activity measured as radical scavenging activity (DPPH) is typically about 16-17 TEAC g/L.

### Spray-dried lingonberry powder

Lingonberries (*Vacciniun vitis-idaea*) are known to contain e.g. quercetin and kaempferol glycosides among other phenolic compounds. The phenolic composition of lingonberries has been widely studied but it is not yet characterized thoroughly. The amounts of different compounds are known to vary according to many factors, such as the growing environment. The conjugated structures also cause additional challenge to analyzing. The main classes of phenolic compounds found in lingonberries are hydroxybenzoic acids, hydroxycinnamic acids, anthocyanins, flavonols, flavan-3-ols and proanthocyanidins. Quercetin has numerous advantageous characteristics, e.g. antioxidative and anti-inflammatory properties. Quercetin is categorized as a flavonol by structure. Flavonols as well as phenolic compounds in general are present in many fruits, berries and vegetables and they mostly appear as conjugated glycosides, i.e. bound to various sugar molecules.

Spray-dried lingonberry powder is typically obtained by pressing the juice from lingonberry fruit, filtering and mixing the juice with maltodextrin, and then spray-drying the mixture. Lingonberries (*Vacciniun vitis-idaea*) are collected from a Nordic forest, typically in northern Finland. The berries are typically heat-treated and treated with pectinase, and the juice is pressed using a conventional presser. Subsequently, the juice may be filtered, pasteurized and concentrated. If necessary, the concentrate is diluted with water, followed by mixing with maltodextrin, for example in a ratio of 45% of lingonberry juice and 55% of maltodextrin (per weight) when 45% lingonberry powder is prepared. The mixture is spray-dried in a conventional spray-drier, by leading the concentrated mixture into the drying chamber as spray, whereby water is evaporated and the dried particles separated. The powder is cooled down, sieved out and packed for further use.

The composition of the spray-dried lingonberry powder (45%) typically consists of mainly carbohydrates 89 g/100g of which about 22 g are sugars, while it also contains approximately 4% of dietary fiber. Total content of polyphenolic compounds varies but is typically 23-30 g/kg, measured using Folin-Ciocalteau method. In the present context, spray-dried lingonberry powder typically has a total quercetin concentration of at least 0.4 mg/g dry matter and/or a total phenolic content of at least 10 mg/g dry matter.

In some embodiments, spray-dried cloudberry powder, spray-dried lingonberry powder, or both may be included in various cosmetic compositions, such as day creams, night creams, eye creams, moisturizers, face fresheners, serums, ampoules, toners, skin lotions, solid products like sticks, cloth faces, mask products, cleanser formulations, facial makeup and color cosmetics, hair products (wash and care products, styling products, masks) or the like, for use in a method of the invention.

The cosmetic compositions may also include any one of or any combination of the following additional cosmetically acceptable ingredients: water, moisturizing agents, preservatives, thickening agents, structuring agents, oils, vitamins, antioxidants, emulsifiers, waxes, fragrances, solubilizants and other cosmetically acceptable ingredients as known by persons skilled in the art and as appropriate for each type of various cosmetic compositions.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

### EXPERIMENTAL

The effect of lingonberry spray-dried powder and cloudberry spray-dried powder was evaluated on the growth of a mix of bacterial strains, *Staphylococcus aureus (S. aureus), Staphylococcus epidermidis (S. epidermidis), Cutibacterium acnes (C. acnes)* and *Corynebacterium xerosis* (*C. xerosis*)*.*

In the first step, a preliminary cytotoxicity assay was performed in order to determine the 2 concentrations to be tested for each compound. The effect of the test compounds was evaluated after 48 hours of incubation with the mix of bacterial strains using an XTT (tetrazolium salt) reduction assay.

In the second part, the effect of the test compounds on bacterial growth was evaluated after 6, 24 and 48 hours of incubation with the mix of bacterial strains. Bacterial enumeration was performed using qPCR technology.

**Table 1. Bacterial strains**

| **Bacterial strain** | **Gram** | **Liquid medium** | **Solid medium** | **Mode of respiration** |
|---|---|---|---|---|
| ***Staphylococcus aureus*** ATCC^{®} 6538^{™} **(*S. aureus*)** | + | / | Mueller Hinton (MH) agar | Aerobic |
| ***Staphylococcus epidermidis*** ATCC^{®} 14990^{™} **(*S. epidermidis*)** | + | / | MH agar | Aerobic |
| ***Cutibacterium acnes*** ATCC^{*} 6919^{™} **(*C*. *acnes*)** | + | Brain heart infusion (BHI) with 1% glucose | MH agar with 5% sheep blood | Anaerobic/aerobic tolerant |
| *C**orynebacterium xerosis*** A*TCC^{®} 373^{™} **(C. xerosis)*** | + | / | MH agar with 5% sheep blood | Aerobic |

| | | | | |
|---|---|---|---|---|
| Before each analysis, an amplification of each bacterial strain was performed in liquid and/or solid medium. | | | | |

| **Bacterial strain mix** | **Assay medium** | **Test concentration** | |
|---|---|---|---|
| ***S. aureus* + *S. epidermidis* + *C. acnes* + *C. xerosis*** | Basal Epilife^{®} medium | XTT reduction | 2×10⁶ CFU/ml for each bacterial strain |
| | | Bacterial enumeration | 1.67×10⁷ CFU/ml for each bacterial strain |

**Table 2. Test compounds**

| **Test compound** | **Aspect/Storage** | **Stock solution** | **Test concentrations** | | |
|---|---|---|---|---|---|
| | | | | | |
| **1413021 CLOUDBERRY SPRAY-DRIED POWDER** Ref. 1413021 CLOUDBERRY SPRAY-DRIED POWDER Purity: 30% FD190629-3 | - Powder - Storage: RT | / | XTT reduction | | 8 concentrations from 0.023 to 50 mg/ml |
| | | | Bacterial enumeration | | 1.5 and 15 mg/ml |
| | | | | | |
| 170201 Lingonberry spray-dried powder Batch: 1800400 FD190912-5 | - Powder - Storage: RT | 100 mg/ml (XTT reduction) or 4 mg/ml (Bacterial enumeration) | | XTT reduction | 8 concentrations from 0.023 to 50 mg/ml |
| | | | | Bacterial enumeration | 0.2 and 2 mg/ml |

### Preliminary cytotoxicity assay

After bacterial amplification, for each bacterial strain, a bacterial suspension was prepared using the assay medium and adjusted to an OD₆₀₀ₙₘ (optical density at 600 nm) of 1. A mix of each bacterial strain was then prepared at 2×10⁶ CFU/ml for each strain.

The mix of bacteria was transferred into 96-well plates containing or not (control) the test compounds. The bacteria were then incubated for 48 hours. All experimental conditions were performed in n=3, except the control condition was performed in n=6.

At the end of the incubation time, the bacterial viability was evaluated using an XTT reduction kit according to the supplier's specifications (Roche, Cell Proliferation Kit II (XTT), measurement of the OD at 450 nm with a reference wavelength at 620 nm).

Briefly, bacteria were incubated with XTT (tetrazolium salt) which is reduced in orange water soluble formazan crystals by dehydrogenases of the bacterial cytoplasmic membrane during bacterial aerobic metabolism. This transformation is proportional to the number of living cells and their metabolic activity. The OD was recorded with a microplate reader (VERSAmax, Molecular Devices). Percentage of viability was calculated as follows: viability (%) = (OD sample / OD control) × 100.

### Bacterial growth

After bacterial amplification, for each bacterial strain, a bacterial suspension was prepared using the assay medium and adjusted to an OD₆₀₀ₙₘ (optical density at 600 nm) of 1. A mix of each bacterial strain was then prepared at 1.67×10⁷ CFU/ml for each strain.

The mix of bacteria was transferred into 24-well plates and the test compounds were added or not. The bacteria were then incubated for 6, 24 and 48 hours. All experimental conditions were performed in n=3. In parallel, a control condition at T0 (test conditions with the mix of bacterial strains) was carried out in n=3.

At the end of each incubation time, bacteria were rinsed with a PBS solution, centrifuged and dry frozen at -80°C.

Quantitative PCR. The genomic DNA (gDNA) samples were extracted using NucleoSpin^{®} Tissue kit (Macherey-Nagel) according to the supplier's instructions. The amount of gDNA was evaluated using a spectrophotometer (Nanovue, GE Healthcare).

The PCRs (Polymerase Chain Reactions) were performed using the "LightCycler^{®}" system (Roche Molecular System Inc.) according to the supplier's instructions. Primer sets were used to amplify the fragment corresponding to the bacterial strains:

**Table 3. Primer sets**

| **Gene name** | **Abbreviation** | **Gene Bank** | **DNA bp** |
|---|---|---|---|
| Staphylococcus aureus subsp, aureus strain Piccinini fibrinogen binding protein (fib) gene, complete cds | Fib | HM014113 | 210 |
| Staphylococcus epidermidis 16S ribosomal RNA (16S RRNA) gene | 16S rRNA | L37605 | 78 |
| Propionibacterium acnes gene for 16S rRNA | 16S rRNA | AB041616 | 167 |
| Corynebacterium xerosis small subunit ribosomal RNA sequence | CORSSRNA | M59058 | 100 |

The reaction mix (10 µl final) was prepared as follows: 2.5 µl of gDNA, primers and TaqMan^{™} probe, and reagent mix containing taq DNA polymerase and MgCl₂. The analysis of transcripts was performed in n=2.

The incorporation of fluorescence in amplified DNA was continuously measured during the PCR cycles. The number of cycles was determined from the "output point" (Ct) of the fluorescence curve (the "output point" is the cycle number for which the fluorescence is significantly different of background noise). For a considered marker, the highest is the cycle number; the lowest is the DNA quantity (a high number of cycle (>30) show a low relative expression, near the limit of detection).

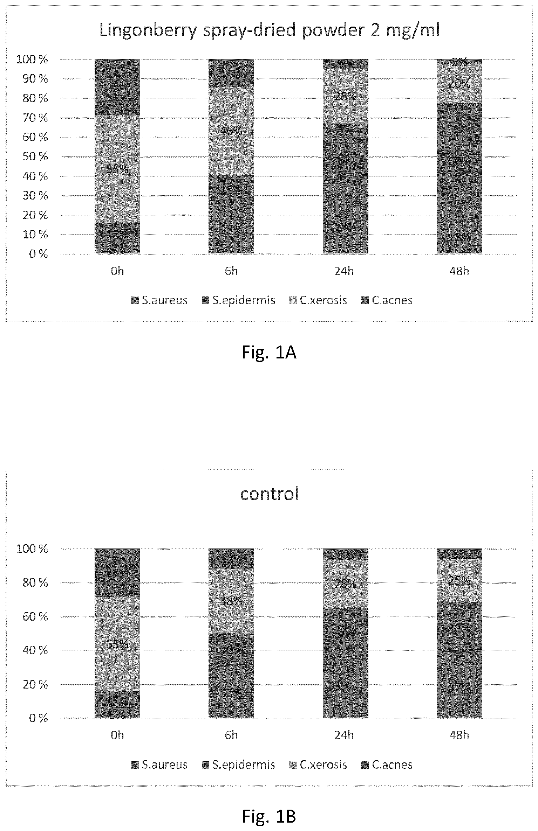

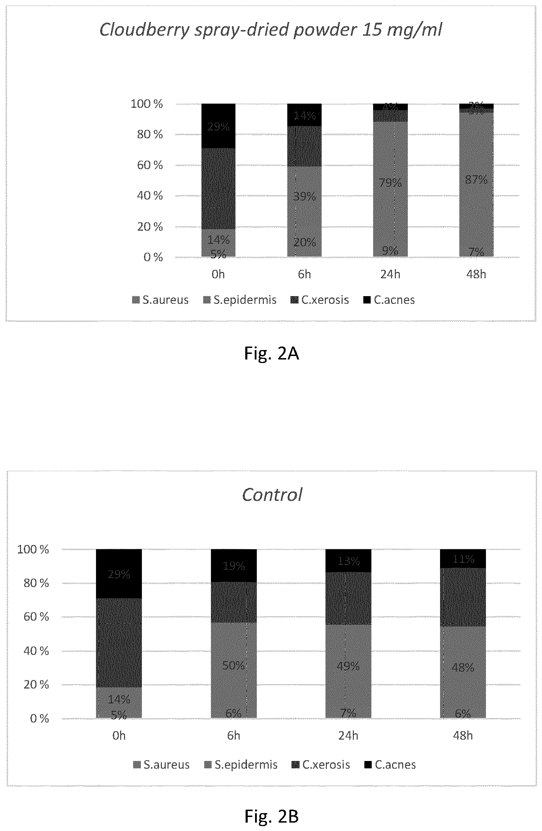

In parallel, standard curves (one for each bacterial strain) were performed using different dilutions of each bacterial suspension in order to establish the correlation between the bacterial quantity and the number of PCR cycles. The quantification of each bacterial strain was determined against the standard curve. The results are shown in Figures 1 and 2.

As can be seen from Figures 1A and 1B, lingonberry spray-dried powder (45%) at a concentration 2 mg/ml remarkably increased the proportion of *S. epidermidis* in relation to *C. acnes* already within 6 hours. Within 48 h, the ratio of *S. epidermidis* to the combined amount of *C. acnes* and *S. aureus* had increased from about 1:3 to about 3:1. At the same time, the ratio of *S. epidermidis* to the combined amount of *C. acnes* and *S. aureus* in the untreated control was about 1:1.3. Moreover, in the control the share of pathogenic *S. aureus* had significantly increased compared to the time point 0 h.

Figures 2A and 2B show that spray-dried cloudberry powder (30%) at a concentration of 15 mg/ml significantly increased the proportion of *S. epidermidis* in relation to *C. acnes* and *S. aureus* already within 24 hours. Within 48 h, the ratio of *S. epidermidis* to the combined amount of *C. acnes* and *S**.** aureus* had increased from about 1:2.5 to about 9:1. At the same time, the ratio of *S**.** epidermidis* to the combined amount of *C. acnes* and *S**.** aureus* in the untreated control was about 2.8:1.

### Formulation Examples

### Example 1: Skin care cream, lotion or gel

| INGREDIENTS | Max. levels % (w/w) |
|---|---|
| Oils, waxes and fats | 30 |
| Humectants | 20 |
| Thickeners | 12 |
| Spray dried berry powder | 0.01-20 |
| Bulking agents | 5 |
| UV filters | 5 |
| Emulsifying agents | 5 |
| Preservatives | 2 |
| Colorants | 2 |
| Perfume | 1 |
| Aqua | to 100 |

### Example 2. Zinc oxide based cream

| INGREDIENTS | Max. levels % (w/w) |
|---|---|
| Oils, waxes and fats | 60 |
| Bulking agents | 30 |
| Zinc oxide | 25 |
| Humectants | 20 |
| Emulsifying agents | 12 |
| Spray-dried berry powder | 0.01-20 |
| Thickeners | 5 |
| Preservatives | 1.5 |
| Perfume | 1 |
| Aqua | to 100 |

### Example 3. Tonic cosmetic water

| INGREDIENTS | Max. levels % (w/w) |
|---|---|
| Humectants | 25 |
| Spray-dried berry powder | 0.01-20 |
| Oils (e.g. vegetable oils) | 50 |
| Additional ingredients | 5 |
| Emulsifying agents | 5 |
| Thickeners | 2 |
| Preservatives | 2 |
| Perfume | 1.5 |
| Colorants | 1 |
| Aqua | to 100 |

### Example 4. Lip care product or lip colour product (stick, cream or gel)

| INGREDIENTS | Max. levels % (w/w) |
|---|---|
| Oils, waxes and fats | 90 |
| Humectants | 50 |
| Aqua | 50 |
| Film forming agents | 30 |
| Thickeners | 15 |
| UV filters | 15 |
| Bulking agents (e.g. silica) | 10 |
| Emulsifying agents | 5 |
| Cosmetic colorants | 5 |
| Spray dried berry powder | 0.01-20 |
| Perfume | 2 |
| Preservatives, antimicrobials | 1 |
| Flavouring agents | 1 |

### Example 5. Cleansing gel

| INGREDIENTS | Max. levels % (w/w) |
|---|---|
| Anionic surfactants | 20 |
| Oils, waxes and fats | 20 |
| Humectants | 20 |
| Non-ionic / amphoteric surfactants | 15 |
| Foam boosting agents | 5 |
| Spray-dried berry powder | 0.01-20 |
| Thickeners | 5 |
| Preservatives | 2 |
| Perfume | 1 |
| Colorants | 1 |
| Aqua | to 100 |

### Example 6. Foundation

| INGREDIENTS | Max. levels % (w/w) |
|---|---|
| Isoparaffin and volatile emollients, like C15-19 alkanes and C13-alkanes | 60 |
| Oils | 40 |
| Emollients (e.g. plant esters) | 30 |
| Silicones | 30 |
| Bulking agents | 30 |
| Colorants, color additives | 30 |
| Humectants | 25 |
| Thickeners, emulsifying agents | 20 |
| UV filters | 10 |
| Surfactants | 5 |
| Spray-dried berry powder | 0.01-20 |
| Perfume | 1 |
| Preservatives | 1 |
| Aqua | to 100 |

### Example 7. Mascaras

| INGREDIENTS | Max. lev % (w/w) |
|---|---|
| Silicones excl. volatile silicones | 80 |
| Oils, waxes and fats | 30 |
| Resins, film forming polymers | 30 |
| Colorants, colour additives | 25 |
| Emulsifying agents (e.g. stearates) | 20 |
| Bulking agents | 15 |
| Thickeners | 12 |
| Aqua | 60 |
| Humectants | 5 |
| Spray-dried berry powder | 0.01-20 |
| Perfume | 1 |
| Antioxidants | 1 |
| Preservatives | 1 |

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

At least some embodiments of the present invention find industrial application in cosmetic industry.

### ACRONYMS LIST

- CFU: colony forming unit
- gDNA: genomic deoxyribonucleic acid
- OD: optical density
- qPCR: quantitative polymerase chain reaction
- rRNA: ribosomal ribonucleic acid
- XTT: 2,3-bis(2-methoxy-4-nitro-5-sulfophenyl)-2H-tetrazolium-5-carboxanilide

### CITATION LIST

### Patent Literature

EP 1337224
EP 2914242
EP 3365068
US 8501246
WO 2006/134583
WO 2018/115582 A1
Non Patent Literature
Byrd A, Belkaid Y, Segre J. (2018) The human skin microbiome. Nat Rev Microbiol 16, 143-155
Callewaert C, Kerckhof F-M, Granitsiotis MS, Van Gele M, Van de Wiele T, et al. (2013) Characterization of Staphylococcus and Corynebacterium Clusters in the Human Axillary Region. PLoS ONE 8(8)
Davis CP. (1996) Chapter 6. Normal Flora. In Baron S (ed.) Medical Microbiology, 4th ed. Galveston. Texas
Iwase T, Uehara Y, Shinji H, Tajima A, Seo H, Takada K, Agata T, Mizunoe Y. (2010). Staphylococcus epidermidis Esp inhibits Staphylococcus aureus biofilm formation and nasal colonization. Nature 465, 346-349
Oates A & McBain AJ (2016) Growth of MRSA and Pseudomonas aeruginosa in a fine-celled foam model containing sessile commensal skin bacteria, Biofouling, 32:1, 25-33
SanMiguel A, Grice EA. (2015) Interactions between host factors and the skin microbiome. Cell Mol Life Sci. 2015;72(8):1499-1515
Wang Y, Kuo S, Shu M, Yu J, Huang S, Dai A, Two A, Gallo RL, Huang CM. (2014). Staphylococcus epidermidis in the human skin microbiome mediates fermentation to inhibit the growth of Propionibacterium acnes: Implications of probiotics in acne vulgaris. Appl Microbiol Biotechnol. 98(1): 411-424

## Claims

1. Non-therapeutic use of spray-dried berry powder from Nordic berries in a cosmetic composition for increasing the ratio of commensal bacteria to harmful or pathogenic bacteria in human skin microbiome.

2. The use according to claim 1, wherein the cosmetic composition comprises spray-dried powder obtained from berries selected from the group consisting of lingonberry *(Vaccinium vitis-idaea),* members of *Rubus* genus, such as cloudberry *(Rubus chamaemorus),* raspberry (*Rubus idaeus*)*,* and Arctic bramble (*Rubus arcticus*)*,* cranberry (*Vaccinium oxycoccus* / *Oxycoccus palustris*)*,* strawberry (*Fragararia x ananassa*) and any combinations thereof.

3. The use according to claim 1 or 2, wherein the cosmetic composition comprises spray-dried lingonberry *(Vaccinium vitis-idaea)* powder, spray-dried cloudberry *(Rubus chamaemorus)* powder, or both, together with a cosmetically acceptable carrier.

4. The use according to any one of the preceding claims for increasing the ratio of *Staphylococcus epidermidis* to harmful or pathogenic bacteria in human skin microbiome.

5. The use according to any one of the preceding claims for increasing the ratio of *Staphylococcus epidermidis* to *Cutibacterium acnes (Propionibacterium acnes), Staphylococcus aureus, Pseudomonas aeruginosa* or other harmful or pathogenic bacteria or any combinations thereof, in particular for increasing the ratio of *S. epidermidis* to *C. acnes, S. aureus* or their combination, more particularly for increasing the ratio of *S. epidermidis* to *S*. *aureus.*

6. The use according to claim 5 for increasing the ratio of *S. epidermidis* to the combined amount of *C. acnes* and *S. aureus,* typically from 1:2.5 - 1:3 to about 3:1 - 10:1 within 48 hours, preferably from about 1:2.5 to at least 3:1 within 24 hours from applying the cosmetic composition on the human skin.

7. The use according to claim 6 for increasing the ratio of *S. epidermidis* to the combined amount of *C. acnes* and *S. aureus* from 1:2.5 - 1:3 to about 3:1 - 10:1 within 48 hours from applying the cosmetic composition on the human skin.

8. The use according to any one of the preceding claims, wherein the cosmetic composition comprises spray-dried lingonberry powder having a lingonberry concentration of at least 30% by weight, preferably at least 45% by weight, and wherein the amount of the spray-dried lingonberry powder in the cosmetic composition is 0.01 to 20 % by weight, preferably 0.05 to 10% by weight, more preferably 0.05 to 5% by weight.

9. The use according to any one of the preceding claims, wherein the cosmetic composition comprises spray-dried cloudberry powder having a cloudberry concentration of at least 30% by weight, preferably at least 40% by weight, and wherein the amount of the spray-dried cloudberry powder in the cosmetic composition is from 0.01 to 20 % by weight, preferably from 0.05 to 10 % by weight, more preferably from 0.05 to 5% by weight.

10. The use according to any one of the preceding claims, wherein the spray-dried berry powder has been obtained by a process comprising the steps of pressing juice from the Nordic berries, filtering and mixing the juice with maltodextrin and then spray-drying the mixture.

11. A non-therapeutic cosmetic method for increasing the ratio of commensal bacteria to harmful or pathogenic bacteria in human skin microbiome, the method comprising applying on the skin a topical cosmetic composition comprising an effective amount of spray-dried berry powder from Nordic berries and a cosmetically acceptable carrier.

12. The method according to claim 11, wherein the cosmetic composition comprises spray-dried lingonberry *(Vaccinium vitis-idaea)* powder, spray-dried cloudberry *(Rubus chamaemorus)* powder, or both.

13. The method according to claim 11, wherein the topical composition comprises spray-dried lingonberry powder, spray-dried cloudberry powder, or both, together with at least one cosmetically acceptable carrier, excipient or other ingredient.

14. The method according to any one of claims 11 to 13, wherein the topical composition comprises spray-dried lingonberry powder, spray-dried cloudberry powder or both in an amount of 0.01 to 20% by weight, preferably from 0.05 to 10% by weight, and wherein the spray-dried lingonberry powder has a lingonberry concentration of at least 45% by weight, and the spray-dried cloudberry powder has a cloudberry concentration of at least 30% by weight.

15. The method according to any one of claims 11 to 14 for increasing the ratio of S. *epidermis* to *C. acnes* and *S. aureus* in human skin microbiome, wherein the ratio of S. *epidermidis* to the combined amount of *C. acnes* and *S. aureus* is increased from about 1:2.5 - 1:3 to about 3:1 - 10:1 within 48 hours, preferably from about 1:2.5 to at least 3:1 within 24 hours from applying the cosmetic composition on the human skin.
